# EUROPÄISCHE PATENTANMELDUNG

(11) **EP 1 484 610 A2**
(43) Veröffentlichungstag der Anmeldung: **08.12.2004**
(21) Anmeldenummer: 04009761.0
(22) Anmeldetag: 24.04.2004
(51) Int. Cl.: G01N 33/00

(54) **Abgasmessvorrichtung**

(30) Priorität: 06.06.2003 DE 10325702
(71) Anmelder: M & C Products Analysentechnik GmbH, 40885 Ratingen (DE)
(72) Erfinder: Benz, Bernhard, 78267 Aach (DE)
(74) Vertreter: Berkenbrink, Kai-Oliver

(57) **Zusammenfassung**

Die Erfindung betrifft eine Abgasmessvorrichtung mit einer Gasentnahmevorrichtung, einer Gasanalysevorrichtung und wenigstens einem Gasführungsorgan, durch das Abgas entlang eines Gasweges von der Gasentnahmevorrichtung zur Gasanalysevorrichtung leitbar ist.

## Beschreibung

Die Erfindung betrifft eine Abgasmessvorrichtung mit einer Gasentnahmevorrichtung, einer Gasanalysevorrichtung und wenigstens einem Gasführungsorgan, durch das Abgas entlang eines Gasweges von der Gasentnahmevorrichtung zur Gasanalysevorrichtung leitbar ist.

Abgase, insbesondere beispielsweise Abgase aus Kohlekraftwerken und Müllverbrennungsanlagen, enthalten häufig Anteile an Quecksilber. Das Quecksilber kann im Abgas entweder elementar - als Hg (0) - oder in Form einer chemischen Verbindung, insbesondere beispielsweise in Form von HgCl₂ vorliegen.

Aufgrund Ihrer Toxizität liegen gesetzliche Grenzwerte für die Anteile von Quecksilber und Quecksilberverbindungen in Abgasen vor.

Entsprechend müssen die Anteile an Quecksilber in Abgasen ständig analysiert und überwacht werden.

Zur Durchführung entsprechender Analysen ist es bekannt, dem Abgas mittels einer Gasentnahmevorrichtung definierte Abgasanteile zu entnehmen und einer Gasanalysevorrichtung, in der diese Abgasanteile analysierbar sind, zuzuleiten.

Aufgrund der in einem Abgas regelmäßig vorhandenen Anteile an sauren Bestandteilen und Schwefeloxiden (HCl, SO₂ SO₃) treten bei der vorbeschriebenen Messmethode jedoch erhebliche Probleme auf.

Denn soweit zur Bestimmung des Quecksilbers Gasanalysevorrichtungen verwendet werden, in denen Quecksilber photometrisch gemessen wird, können die Anteile an Schwefeloxiden im gemessenen Gas das Absorptionsspektrum des Quecksilbers überlagern, was zu Fehlmessungen bei der Bestimmung des Quecksilberanteils führen kann.

Darüber hinaus können die Schwefeloxide zusammen mit weiteren Bestandteilen des Abgases - insbesondere wenn dieses seine Taupunkttemperatur unterschreitet - Säuren bilden, die die Abgasmessvorrichtung beschädigen können.

Der Erfindung liegt die Aufgabe zugrunde, eine Abgasmessvorrichtung zur Verfügung zu stellen, durch die negative Einwirkungen, insbesondere Interferenzen von im Abgas vorhandenen Schwefeloxiden sowie sauren Gasbestandteilen auf die Messvorrichtung verhindert werden können.

Zur Lösung dieser Aufgabe wird eine Abgasmessvorrichtung mit in ihrer allgemeinsten Ausführungsform folgenden Merkmalen vorgeschlagen:
- Einer Gasentnahmevorrichtung,
- einer Gasanalysevorrichtung und
- wenigstens einem Gasführungsorgan, durch das Abgas entlang eines Gasweges von der Gasentnahmevorrichtung zur Gasanalysevorrichtung leitbar ist, wobei
- der Gasweg durch wenigstens eine Aluminiumoxid-Schüttung geführt ist.

Der Erfindung liegt die folgende Erkenntnis zugrunde:

Wenn der zu analysierende Abgasstrom - vor dessen Analysierung in der Gasanalysevorrichtung - durch eine Aluminiumoxid-Schüttung geführt wird, werden die im Abgas vorhandenen Anteile an Schwefeloxiden sowie die sauren Gasanteile durch das Aluminiumoxid adsorbiert, so dass diese Schwefeloxidanteile und sauren Gasanteile, nach deren Durchgang des Abgases durch die Aluminiumoxid-Schüttung, keine negativen Einwirkungen auf die Abgasmessvorrichtung bzw. die Analysierung des Abgases ausüben können.

Es sind Verfahren bekannt, bei denen Aluminiumoxid zur Trocknung von Gasen verwendet werden kann.

In der anmeldungsgemäßen Abgasmessvorrichtung liegt das Aluminiuoxid (Al₂O₃) in Form einer Schüttung vor, also als Granulat. Dieses Granulat kann eine hohe offene Porosität und damit eine hohe spezifische Oberfläche besitzen, um die Adsorption von zu adsorbierenden Gasbestandteilen zu verbessern. Die spezifische Oberfläche des verwendendeten Aluminiumoxides kann beispielswesie zwischen 250 und 450 m²/g liegen, also beispielsweise auch in einem Bereich zwischen 300 und 400 m²/g oder zwischen 350 und 380 m²/g .

Die Dichte des Aluminiumoxides kann beispielsweise unter 1 g/cm³ liegen, also beispielsweise zwischen 0,6 und 0,8 g/cm³ oder zwischen 0,65 und 0,75 g/cm³.

Besondere Bedeutung kommt auch dem für die Schüttung verwendeten Kornband des Aluminiumoxides zu: Würde zu feines Aluminiumoxid verwendet werden (Durchmesser beispielsweise unter 0,5 mm), könnte das Gas nicht mehr durch die Schüttung geführt werden, da die Schüttung zu dicht wäre; würde zu grobes Aluminiumoxid verwendet werden (Durchmesser beispielsweise über 10 mm) wäre die Reaktionsoberfläche zu gering, so dass kein ausreichender Adsorptiongrad ereicht würde. Das Kornband (Durchmesser des Aluminiumoxid-Granulats) kann anmeldungsgemäß beispielsweise im Bereich zwischen 0,5 und 10 mm liegen, also beispielsweise auch im Bereich zwischen 0,5 und 8 mm oder zwischen 2 und 8 mm.

Als besonders geeignetes Aluminium für die Anforderungen in der anmeldungsgemäßen Abgasmessvorrichtung hat sich sogenanntes "aktiviertes Aluminium" erwiesen.

Nach einer erfindungsgemäßen Ausführungsform ist wenigstes einer Aluminiumoxid-Schüttung eine Heizvorrichtung, durch die das durch die Aluminiumoxid-Schüttung leitbare Gas erhitzbar ist, zugeordnet. Insbesondere kann die Heizvorrichtung derart ausgelegt sein, dass durch sie das durch die Aluminiumoxid-Schüttung leitbare Gas auf eine Temperatur erhitzbar ist, die über der Taupunkttemperatur des Gases liegt. Indem das durch die Aluminiumoxid-Schüttung geleitete Gas entsprechend erhitzt wird, kann verhindert werden, dass das Abgas - bzw. irgendeines der Gase, aus denen sich das Abgas zusammensetzt - im Bereich der Aluminiumoxid-Schüttung seinen Taupunkt unterschreitet. Würde das Abgas - wenn es nicht erhitzt würde - an irgendeinem Punkt der Abgasmessvorrichtung, insbesondere beispielsweise im Bereich der Aluminiumoxid-Schüttung, seinen Taupunkt unterschreiten, käme es dort zur Bildung von Kondensflüssigkeit. Würden etwaig zu analysierende Abgas-Bestandteile jedoch in der Kondensflüssigkeit gebunden sein, hätte dies zwangsläufig eine Verfälschung der Abgasanalyse in der Gasanalysevorrichtung zur Folge. Weiterhin könnten sich als Kondensflüssigkeit auch Säuren bilden, die Bestandteile der Abgasmessvorrichtung beschädigen könnten.

Die vorgenannten Nachteile können durch die erfindungsgemäße Heizvorrichtung mithin verhindert werden. Die Heizvorrichtung kann beispielsweise unmittelbar im Bereich der Aluminiumoxid-Schüttung angeordnet sein, kumulativ oder alternativ aber beispielsweise auch im Bereich der Gasführungsorgane, die zwischen Gasentnahmevorrichtung und Aluminiumoxid-Schüttung angeordnet sind oder beispielsweise auch im Bereich der Gasentnahmevorrichtung oder in der Gasentnahmevorrichtung selbst.

Die Heizvorrichtung kann beispielsweise derart ausgelegt sein, dass durch sie das durch die Aluminiumoxid-Schüttung leitbare Gas auf eine Temperatur von über 90° C erhitzbar ist, also beispielsweise auch über 140° C oder über 160° C. Dabei kann die Heizvorrichtung derart ausgelegt sein, dass durch sie das durch die Aluminiumoxid-Schüttung leitbare Gas auf eine Temperatur in einem Temperaturintervall zwischen 90°C und 320°C erhitbar ist, also bespielsweise auch in einem Temperaturintervall zwischen 140°C und 220°C:

Um zu verhindern, dass das Abgas in irgendeinem Bereich der Abgasmessvorrichtung seinen Taupunkt unterschreitet, können - neben einer etwaig der Aluminiumoxid-Schüttung zugeordneten Heizvorrichtung - der Abgasmessvorrichtung eine oder mehrere der zuvor beschriebenen Heizvorrichtungen zugeordnet sein, so dass gewährleistet ist, dass das Abgas seinen Taupunkt in keinem Bereich der Abgasmessvorrichtung unterschreitet.

Alternativ oder kumulativ zu den vorgenannten Heizvorrichtungen kann die anmeldungsgemäße Abgasmessvorrichtung mit einem oder mehreren Gaskühlern ausgerüstet sein. Durch diese Gaskühler ist das Abgas abkühlbar und damit auf eine definierte (erniedrigte) Taupunkttemperatur des Abgases einstellbar.
Dabei kann beispielsweise vorgesehen sein, dass das Abgas derart abgekühlt wird, dass seine Taupunkttemperatur so niedrig ist, dass das Abgas auf seinem strömungstechnisch hinter dem Gaskühler liegenden Gasweg seine Taupunkttemperatur nicht unterschreitet. Beispielsweise kann vorgesehen sein, das Abgas durch den Gaskühler auf eine Taupunkttemperatur unter 10 °C abzusenken, beispielsweise auf eine Taupunkttemperatur zwischen 1°C und 10 °C, also beispielsweise auch auf eine Taupunkttemperatur zwischen 2°C und 8 °C oder zwischen 3 °C oder 7 °C.

Der Gaskühler kann grundsätzlich an einer beliebigen Stelle des Gasweges angeordnet sein. Nach einer Ausführungsform ist vorgesehen, den Gaskühler strömungstechnisch unmittelbar hinter der Gasentnahmevorrichtung anzuordnen dadurch wird die Taupunkttemperatur auf dem gesamten Gasweg auf einen definerten Wert gesenkt. Nach einer anderen Ausführungsform ist vorgesehen, den Gaskühler strömungstechnisch unmittelbar vor der Gasanalysevorrichtung anzuordnen - dadurch wird die Taupunkttemperatur in der Gasanalysevorrichtung auf einen definierten Wert gesenkt.

Durch die Verwendung eines oder mehrerer vorgenannten Gaskühler ist es möglich, auf die vorgenannten Heizvorrichtungen zu verzichten.

Die Aluminiumoxid-Schüttung kann in einem gasdichten Körper, der über wenigstens einen Gaseinlass und wenigstens einen Gasauslass mit den Gasführungsorganen verbunden ist, angeordnet sein.

Dieser Körper kann beispielsweise aus einem Metall- oder einem Kunststoffmantel oder einer Kombination daraus gebildet sein. Im Körper kann ein Halteorgan angeordnet sein, auf oder in dem die Aluminiumoxid-Schüttung gehaltert ist. Bei diesem Halteorgan kann es sich beispielsweise um ein Sieb, ein Lochblech, einen Filter oder einer Kombination daraus handeln, auf dem die Aluminiumoxid-Schüttung aufgeschüttet ist oder in denen die Aluminiumoxid-Schüttung - wie in einem "Teesieb" - angeordnet ist.

Durch die Gasentnahmevorrichtung ist ein Abgas, beispielsweise ein Abgas eines Kraftwerkes, in die Abgasmessvorrichtung einleitbar.

Als Gasentnahmevorrichtung kann beispielsweise eine sogenannten "Gasentnahmesonde" verwendet werden. Eine solche Gasentnahmesonde weist ein Entnahmerohr auf, das in ein Abgas einführbar ist. Über die das Entnahmerohr kann Abgas durch die Gasentnahmevorrichtung aufgenommen und anschließend in die Abgasmessvorrichtung eingeleitet werden. Das Entnahmerohr kann mit einem Gas-Vorfilter kombiniert sein. Zur Verhinderung der Taupunktunterschreitung des Abgases in der Gasentnahmevorrichtung kann dieser eine der oben beschriebenen Heizvorrichtungen zugeordnet sein.

Durch die Gasanalysevorrichtung ist ein Gas, hier ein Abgas, insbesondere chemisch und physikalisch analysierbar. Bei der Gasanalysevorrichtung kann es sich insbesondere um eine solche Vorrichtung handeln, mit der auch die Quecksilber- und Chloranteile eines Gases analysierbar sind. Beispielsweise kann es sich um eine Vorrichtung handeln, mit der die Bestandteile eines Gases spektrophometrisch messbar sind.

Mittels des oder der Gasführungsorgane ist das von der Gasentnahmevorrichtung aufgenommene Abgas entlang eines Gasweges von der Gasentnahmevorrichtung zur Gasanalysevorrichtung leitbar.

Bei den Gasführungsorganen kann es sich beispielsweise um Schläuche oder Rohrleitungen handeln, durch die ein Gas leitbar ist.

Das durch die Gasanalysevorrichtung im Abgas zu analysierende Quecksilber liegt im Abgas in der Regel nicht in elementarer Form - als Hg (0) - vor, sondern in Form von Verbindungen, insbesondere in Form von HgCl₂.

Um dieses, an das Chlor gebundene Quecksilber im Abgas analysieren zu können, ist es notwendig, zumindest einen Teil des HgCl₂ vor der Analyse des Abgases in der Gasanalysevorrichtung in seine Quecksilber- und Chlor-Bestandteile aufzuspalten.

Zur Aufspaltung der in einem Abgas vorhandenen chemischen Verbindungen in ihre jeweiligen Grundbestandteile sind sogenannte Konverter bekannt. In diesen Konvertern werden - üblicherweise in Gegenwart eines geeigneten Katalysators - chemische Verbindungen in ihre Grundbestandteile aufgespalten. Zur Beschleunigung der Katalyse ist auch bekannt, diese Katalysatoren zu erhitzen.

Der Konverter kann dazu mit einer Heizvorrichtung versehen sein, durch die der Katalysator auf eine Temperatur zwischen 350° C und 800° C erhitbar ist, also beispielsweise auch auf eine Temparatur zwischen 500° C und 750° C oder zwischen 600° C und 700° C.

Diese Aufspaltung kann im Konverter beispielsweise in Form einer Pyrolyse erfolgen. Beispielsweise ist bekannt, HgCl₂ in Gegenwart von Nickel als Katalysator zu pyrolisieren. Ein entsprechendes Verfahren wird in der deutschen Offenlegungsschrift DE 100 45 212 A1 beschrieben.

In den Konverter strömt ein mit Quecksilber und Quecksilberchlorid verunreinigtes Abgas ein. Beim Durchgang durch den Konverter wird das Quecksilber vom Quecksilberchlorid abgespalten. Nach dem Durchgang durch den Konverter weist das Abgas damit nur noch elementares Quecksilber auf.

Bei isolierter Betrachtung eines durch einen entsprechenden Konverter geführten Abgases lässt sich damit nur nachweisen, welche Quecksilberbestandteile das Abgas insgesamt aufweist. Um ebenfalls nachweisen zu können, in welchen Anteilen das Quecksilber im Abgas zum einen in elementarer und zum anderen in Form von Verbindungen vorliegt, ist ein sogenanntes Differenz-Messverfahren bekannt. Bei diesem Differenz-Messverfahren wird das Abgas vor seiner Einleitung in die Abgasanalysevorrichtung in zwei Teil-Abgaswege (Teil-Gaswege) aufgezweigt, von denen jeweils nur einer durch einen Konverter geführt ist. Bei dem durch den Konverter geleiteten Gas ist in der Gasanalysevorrichtung die Gesamtmenge des im Abgas vorhandenen Quecksilbers bestimmbar - also die Summe an Quecksilber in elementarer und verbundener Form. Bei dem nicht durch den Konverter geleiteten Gasstrom ist allein die im Abgas in elementarer Form vorliegende Form von Quecksilber bestimmbar. Durch einen Vergleich der jeweils ermittelten Quecksilberanteile ist bestimmbar, welche Quecksilberanteile im Abgas in elementarer bzw. in verbundener Form vorliegen.

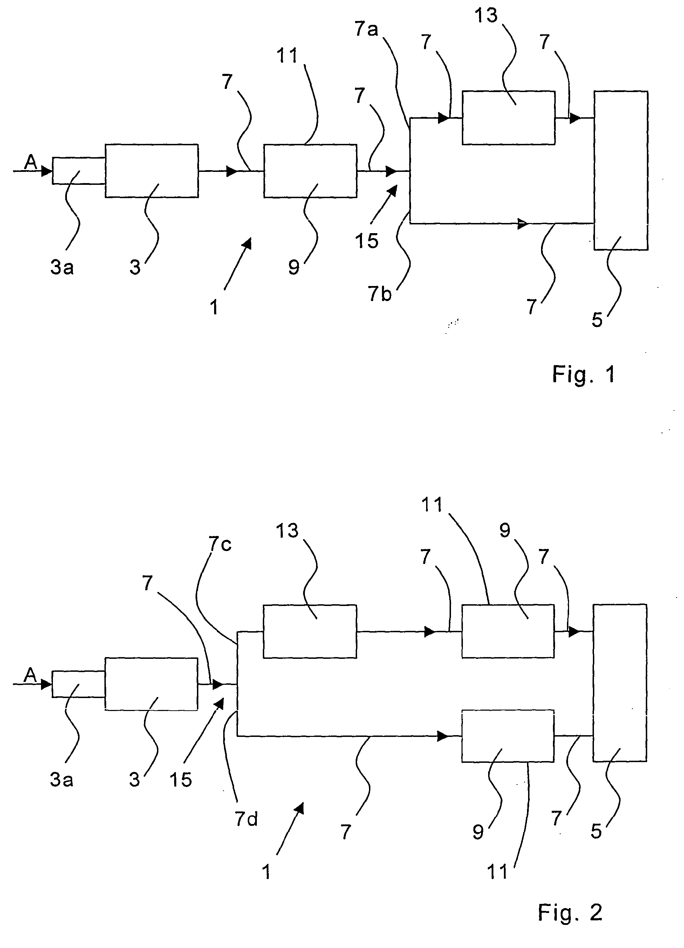

Um ein entsprechendes Differenz-Messverfahren vornehmen zu können, ist bei einer erfindungsgemäßen Ausführungsform der Gasweg zwischen wenigstens einer Aluminiumoxid-Schüttung und der Gasanalysevorrichtung in zwei Teil-Gaswege, entlang derer jeweils ein Teilstrom des Abgases zur Gasanalysevorrichtung leitbar ist, aufgezweigt. Einer dieser Teil-Gaswege kann durch einen Konverter geführt sein (vgl. Fig. 1)

Eine entsprechende Ausführungsform bietet sich insbesondere für die Messung eines Abgases an, das mit hohen Anteilen an Schwefeloxiden verunreinigt ist, denn hier kann das Abgas bereits vor seiner Einleitung in den Konverter in der Aluminiumoxid-Schüttung von Schwefeloxidanteilen befreit werden, so dass diese den Konverter nicht beschädigen können.

Nach einer alternativen Ausführungsform ist vorgesehen, dass der Gasweg zwischen der Gasentnahmevorrichtung und der Gasanalysevorrichtung in zwei Teil-Gaswege, die durch eine jeweilige Aluminiumoxid-Schüttung geführt und entlang derer jeweils ein Teilstrom des Abgases zur Gasanalysevorrichtung leitbar ist, aufgezweigt ist. Bei dieser Ausführungsform kann einer der Teil-Gaswege zwischen der Aufzweigung (also dem Punkt, an dem der Gasweg in zwei Teil-Gaswege aufgezweigt wird) und der Aluminiumoxid-Schüttung, durch die er geführt ist, durch einen Konverter geführt sein (vgl. Fig. 2).

Eine solche Ausführungsform bietet sich beispielsweise bei nur mit gering mit Schwefeloxiden verunreinigten Abgasen an; denn hier wird das Abgas, wie zuvor ausgeführt, zunächst durch den Konverter und erst anschließend durch die Aluminiumoxid-Schüttung geleitet.

Der Vorteil dieser letztgenannten Ausführungsform, bei der Abgas zunächst durch den Konverter und erst anschließend durch die Aluminiumoxid-Schüttung geleitet wird, liegt insbesondere auch darin, dass der Konverter hier unmittelbar benachbart zur Gasentnahmevorrichtung angeordnet werden kann. Dadurch kann für die Gasentnahmevorrichtung (um das Abgas hier über seinen Taupunkt zu erhitzen) und den Konverter (um die Umsetzung des HgCl₂ zu beschleunigen) eine einheitliche Heizvorrichtung vorgesehen sein. Dies spart Raum und Kosten.

Sämtliche der vorgenannten Merkmale der Abgasvorrichtung können einzeln oder in Kombination jeweils beliebig miteinander kombiniert werden.

Weitere Merkmale der Erfindung ergeben sich aus den Unteransprüchen sowie den sonstigen Anmeldungsunterlagen, insbesondere den Figuren.

Zwei erfindungsgemäße Ausführungsbeispiele der Abgasmessvorrichtung sollen anhand der nachfolgenden, stark schematisierten Figuren näher erläutert werden.

Dabei zeigt
- Figur 1: eine Abgasmessvorrichtung, bei der die Aluminiumoxid-Schüttung (entlang des Gasweges) vor dem Konverter angeordnet ist und
- Figur 2: eine Abgasmessvorrichtung, bei der die Aluminiumoxid-Schüttung (entlang des Gasweges) nach dem Konverter angeordnet ist.

Entsprechende Bauteile sind in beiden Figuren jeweils mit den gleichen Bezugszeichen versehen.

Die in Figur 1 insgesamt mit den Bezugszeichen 1 bezeichnete Abgasmessvorrichtung weist eine Gasentnahmevorrichtung 3, eine Gasanalysevorrichtung 5 sowie mehrere, jeweils mit 7 bezeichnete Gasführungsorgane auf.

Die Strömungsrichtung des Abgases beim Durchgang durch die Abgasmessvorrichtung 1 ist jeweils durch Pfeile an den Gasführungsorganen 3 angedeutet.

Die Gasentnahmevorrichtung 3, hier in Form einer Gassonde, weist eine, hier nach links, wegstehendes Gasentnahmerohr 3a mit einem Vorfilter auf, das in ein Abgas führbar ist. Das Abgas wird durch die Sonde 3a aufgenommen - hier durch A kenntlich gemacht - in die Gasentnahmevorrichtung 3 geleitet und von dieser über ein erstes Gasführungsorgan 7 einer Aluminiumoxid-Schüttung 9 zugeleitet.

Das Gasführungsorgan 7 besteht, wie auch die weiteren Gasführungsorgane 7, aus einem Kunststoffschlauch.

Die Aluminiumoxid-Schüttung 9 ist über ein - hier nicht dargestelltes - Halteorgan in Form eines Lochbleches in einem gasdichten Kunststoffgehäuse 11 angeordnet.

Nachdem das aus dem Gehäuse 11 ausgeleitet und über ein Gasführungsorgan 7 ein Stück weitergeleitet worden ist, wird der durch dieses Gasführungsorgan 7 definierte Gasweg an einer Abzweigung 15 in zwei Teil-Gaswege 7a bzw. 7b aufgezweigt.

Beide Teil-Gaswege 7a, 7b werden wiederum durch Gasführungsorgane 7 gebildet.

Während das entlang des Teii-Gasweges 7b geleitete Abgas unmittelbar in die Gasanalysevorrichtung 5 geleitete wird, wird das entlang des Teil-Gasweges 7a geleitete Abgas über einen Konverter 13 in die Gasanalysevorrichtung 5 geleitet.

Im Konverter 13 wird das im Abgas vorhandene HgCl₂ mittels Pyrolyse in Hg (0) und Cl₂ konvertiert.

In der Gasanalysevorrichtung 5 werden die zum einen entlang des Teil-Gasweges 7a und zum anderen die entlang des Teil-Gasweges 7b in die Gasanalysevorrichtung 5 geleiteten Teil-Abgasmengen analysiert und mittels der Durchführung eines Differenz-Messverfahrens festgestellt, welche Anteile an Quecksilber, an Chlor und an Quecksilberverbindungen im Abgas vorhanden sind.

Sowohl der Gasentnahmevorrichtung 3 als auch der Aluminiumoxid-Schüttung 9 ist jeweils eine - in Figur 1 nicht dargestellte - Heizvorrichtung zugeordnet, durch die das durch die Gasentnahmevorrichtung 3 bzw. die Aluminiumoxid-Schüttung 9 strömende Abgas jeweils auf eine Temperatur von 180° Celsius aufheizbar ist.

Der Konverter 13 ist ebenfalls mit einer - nicht dargestellten - Heizvorrichtung versehen, durch die die Konvertierung in diesem bei 650° Celsius vorgenommen werden kann.

Bei der Abgasmessvorrichtung gemäß Figur 2 sind die Gasentnahmevorrichtung 3, die Gasanalysevorrichtung 5, die Gasführungsorgane 7, der Konverter 13 sowie die beiden, jeweils in einem Gehäuse 11 angeordneten Aluminiumoxid-Schüttungen 9 entsprechend der Ausführungsform in Figur 1 ausgebildet, so dass auf deren Aufbau hier nicht erneut näher eingegangen wird.

Der Aufbau der Abgasmessvorrichtung nach Figur 2 ist im Einzelnen wie folgt.

Das durch die Gasentnahmevorrichtung 3 aufgenommene und der Abgasmessvorrichtung 1 zugeleitete Abgas wird - nach Durchleitung des Abgases durch die Gasentnahmevorrichtung 3 - an einer Abzweigung 15 des durch ein Gasführungsorgan 7 definierten Gasweges in zwei Teil-Gaswege 7c, 7d aufgezweigt.

Die Teilabgasmenge, die entlang des Teil-Gasweges 7d geleitet wird, wird zunächst durch eine Aluminiumoxid-Schüttung 9 und, nach dem Durchgang durch diese, der Gasanalysevorrichtung 5 zugeleitet.

Die entlang des anderen Teil-Gasweges 7c geleitete Teil-Abgasmenge wird zunächst durch einen Konverter 13, anschließend durch eine Aluminiumoxid-Schüttung 9 und, nach dem Durchgang durch diese, ebenfalls der Gasanalysevorrichtung 5 zugeleitet.

Die Ausführungsform gemäß Figur 1 bietet sich, wie in der Beschreibung bereits ausgeführt, insbesondere bei stark, die Ausführungsform nach Figur 2 insbesondere bei schwach mit sauren Bestandteilen verunreinigten Abgasen an.

## Patentansprüche

1. Abgasmessvorrichtung mit
a) einer Gasentnahmevorrichtung (3),
b) einer Gasanalysevorrichtung (5) und
c) wenigstens einem Gasführungsorgan (7), durch das Abgas entlang eines Gasweges von der Gasentnahmevorrichtung (3) zur Gasanalysevorichtung (5) leitbar ist,
**dadurch gekennzeichnet, dass**
d) der Gasweg durch wenigstens eine Aluminiumoxid-Schüttung (9) geführt ist.

2. Abgasmessvorrichtung nach Anspruch 1, bei der die Körnung der Aluminiumoxid-Schüttung (9) zwischen 0,5 und 10 mm liegt.

3. Abgasmessvorrichtung nach Anspruch 1, bei der wenigstens einer Aluminiumoxid-Schüttung (9) eine Heizvorrichtung, durch die das durch die Aluminiumoxid-Schüttung (9) leitbare Gas erhitzbar ist, zugeordnet ist.

4. Abgasmessvorrichtung nach Anspruch 3 mit einer Heizvorrichtung, durch die das durch die Aluminiumoxid-Schüttung (9) leitbare Gas auf eine Temperatur erhitzbar ist, die über der Taupunktstemperatur des Gases liegt.

5. Abgasmessvorichtung nach Anspruch 3 mit einer Heizvorrichtung, durch die das durch die Aluminiumoxid-Schüttung (9) leitbare Gas auf über 100°C erhitzbar ist.

6. Abgasmessvorrichtung nach Anspruch 1 mit wenigstens einem Gaskühler, durch die das Abgas abkühlbar ist.

7. Abgasmessvorrichtung nach Anspruch 1, bei der das Abgas derart abkühlbar ist, dass seine Taupunkttemperatur unter 10 °C liegt.

8. Abgasmessvorrichtung nach Anspruch 1, bei der der Gasweg zwischen wenigstens einer Aluminiumoxid-Schüttung (9) und der Gasanalysevorrichtung (5) in zwei Teil-Gaswege (7a, 7b), entlang derer jeweils ein Teilstrom des Abgases zur Gasanalysevorrichtung (5) leitbar ist, aufgezweigt ist.

9. Abgasmessvorrichtung nach Anspruch 6, bei der einer der Teil-Gaswege (7a, 7b) durch einen Konverter (13) geführt ist.

10. Abgasmessvorrichtung nach Anspruch 1, bei der der Gasweg zwischen der Gasentnahmevorrichtung (3) und der Gasanalysevorrichtung (5) in zwei Teil-Gaswege (7c, 7d), die durch eine jeweilige Aluminiumoxid-Schüttung (9) geführt und entlang derer jeweils ein Teilstrom des Abgases zur Gasanalysevorrichtung (5) leitbar ist, aufgezweigt ist.

11. Abgasmessvorrichtung nach Anspruch 10, bei der einer der Teil-Gaswege (7c, 7d) zwischen der Aufzweigung (15) und der Aluminiumoxid-Schüttung (9), durch die er geführt ist, durch einen Konverter (13) geführt ist.
